(19) Europäisches Patentamt
European Patent Office
Office européen des brevets

(11) Veröffentlichungsnummer: **0 366 216 B1**

(12) **EUROPÄISCHE PATENTSCHRIFT**

(45) Veröffentlichungstag der Patentschrift: **12.01.94**

(51) Int. Cl.$^5$: **C07F 7/18**

(21) Anmeldenummer: **89250057.0**

(22) Anmeldetag: **13.10.89**

# (54) (1S,2S,3R,5R)-2-[(3S)-2-Halogen-3-hydroxy-1-alken(inyl)]3-trialkyl-silyloxy-7,7-(2,2-dimethyl-trimethyl--enoxy)-bicyclo[3.3.0]octan-Verbindungen und Verfahren zu deren Herstellung(12.03.90)

(30) Priorität: **13.10.88 DE 3835162**

(43) Veröffentlichungstag der Anmeldung:
**02.05.90 Patentblatt 90/18**

(45) Bekanntmachung des Hinweises auf die Patenterteilung:
**12.01.94 Patentblatt 94/02**

(84) Benannte Vertragsstaaten:
**AT BE CH DE ES FR GB GR IT LI LU NL SE**

(56) Entgegenhaltungen:
**EP-A- 0 234 158**
**EP-A- 0 268 548**

**JOURNAL OF THE AMERICAN CHEMICAL SOCIETY, Band 106, Nr. 13, 27. Juni 1984, Seiten 3875-3876, American Chemical Society; E.J. COREY et al.: "Total Sythesis of C22-Prostanoids in the E and F Series Based on Docosahexaenoic Acid"**

(73) Patentinhaber: **SCHERING AKTIENGESELLSCHAFT Berlin und Bergkamen**
**Müllerstrasse 170/178**
**Postfach 65 03 11**
**D-13303 Berlin(DE)**

(72) Erfinder: **Harre, Michael**
**Fröaufstrasse 8**
**D-1000 Berlin 41(DE)**
Erfinder: **Westermann, Jürgen**
**Elberfelder Strasse 19**
**D-1000 Berlin 21(DE)**

**EP 0 366 216 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von (1S, 2S, 3R, 5R)-2-[(3S)-2-Halogen-3-hydroxy-1-alken(inyl)]-3-trialkylsilyl-oxy-7,7 -(2,2-di methyl-trimethylendioxy)-bicyclo [3.3.0] octan-Verbindungen durch Reduktion von 15-Ketocarbacyclin-Zwischenprodukten (PG-Nomenklatur) mit Diisobutylaluminium-2,6-di-tert.-butyl-4-methyl-phenoxid.

Bei den Synthesen der pharmakologisch wirksamen Carbacyclin-Analoga "Cicaprost"

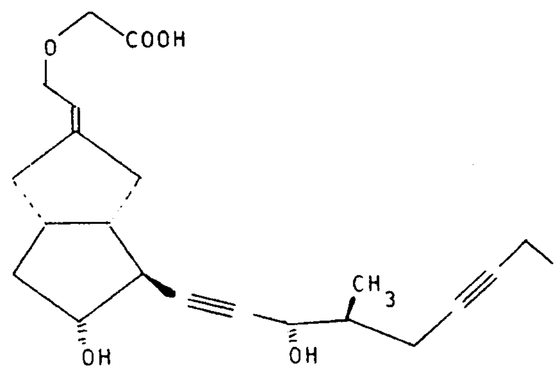

bzw. "Eptaloprost"

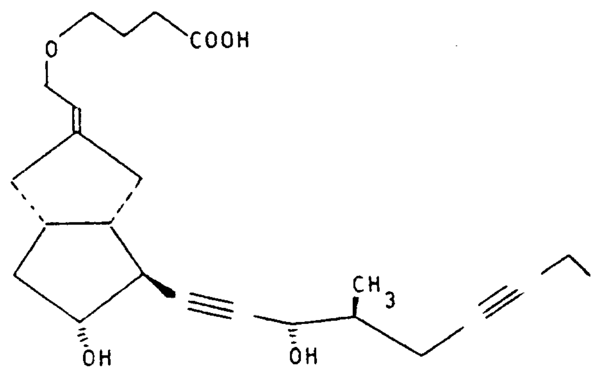

spielt die Reduktion der 15-Ketogruppe zur 15α-Hydroxygruppe auf einer Zwischenstufe der Synthese eine wichtige Rolle. Die Reduktion mit technisch leicht zugänglichen Reagenzien wie Natriumborhydrid führt zu einem Gemisch mit dem unerwünschten 15β-Hydroxy-Epimeren. Die beiden Epimeren müssen durch Chromatographie voneinander getrennt werden. (Aus wirtschaftlichen Gründen muß das 15β-Hydroxyepimere zurückoxidiert und erneut reduziert werden und in die 15-Epimeren getrennt werden usw.).

Der zur Trennung erforderliche Aufwand an Adsorbens und Lösungsmittelmenge ist umso höher, je mehr 15β-Hydroxy-epimeres abgetrennt werden muß. Nach den bisher bekannten Verfahren, die mit einfachen Hydrid-Reagenzien arbeiten, ist ein verhältnismäßig hoher Aufwand zur Herstellung der 15α-Hydroxyepimeren notwendig. Es bestand daher die Aufgabe, die chemische Reduktion der 15-Ketogruppe bei der Synthese von Carbacyclin-Analoga im Hinblick auf die Ausbeute an 15α-Hydroxy-Epimerem weiter zu verbessern.

Die Erfindung betrifft ein Verfahren zur Herstellung von (1S, 2S, 3R,5R)-2-[(3S)-2-Halogen-3-hydroxy-1-alken(inyl)]-3-trialkylsilyl-oxy-7,7-(2,2-dimethyl-trimethylendioxy)-bicyclo-[3.3.0]-octan-Verbindungen der allgemeinen Formel I

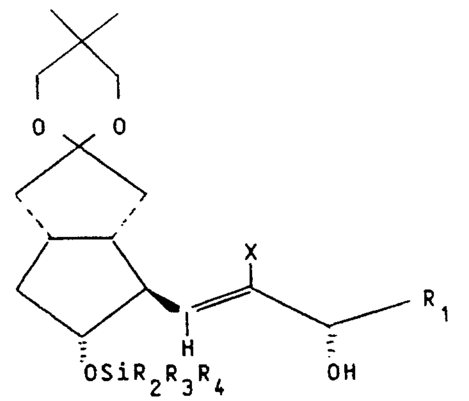

(I),

worin

- X Chlor oder Brom,
- $R_1$ geradkettiges oder verzweigtes Alkyl mit bis zu 6 C-Atomen, geradkettiges oder verzweigtes Alkenyl mit bis zu 6 C-Atomen, geradkettiges oder verzweigtes Alkinyl mit bis zu 6 C-Atomen,
- $R_2$, $R_3$, $R_4$ jeweils gleich oder verschieden sein können und $C_1$-$C_4$-Alkyl und ggf. durch $C_1$-$C_4$-Alkylgruppen substituiertes Phenyl bedeuten können, wobei die 13,14-Doppelbindung bezüglich der C-Kette die trans-Konfiguration aufweist,

durch stereoselektive Reduktion eines α-Halogen-enons der allgemeinen Formel II

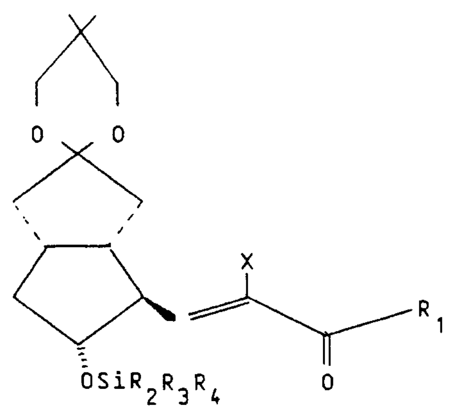

(II),

worin X, $R_1$, $R_2$, $R_3$, $R_4$ die vorstehend angegebene Bedeutung haben, mit einem aus Diisobutylaluminiumhydrid und 2,6-Di-tert.-butyl-4-methyl-phenol hergestellten Reagenz.

Die dargestellten optischen Isomeren besitzen die gleiche relative stereochemische Konfiguration wie das entsprechende Prostacyclin aus Säugetiergewebe oder, im Fall eines Zwischenproduktes, diejenige stereochemische Konfiguration, die ein prostacyclinartiges Produkt mit gleicher relativer stereochemischer Konfiguration wie das Prostacyclin aus Säugetiergewebe hätte.

Ein Hauptproblem bei der Synthese von Carbacyclinen, Prostaglandinen und Prostacyclinen ist die stereoselektive Einführung von funktionellen Gruppen. Besondere Probleme bereitet die stereochemische Kontrolle am C15 in der konformativ beweglichen Seitenkette.

Es ist daher sehr oft erwünscht, das 15α-Hydroxy-epimere bevorzugt gegenüber dem 15β-Hydroxy-epimeren herzustellen, da diese stereochemische Anordnung bei den späteren Umwandlungen in prostacyclinartige Verbindungen nach bekannten Methoden erhalten bleibt. Man erhält somit Verbindungen mit gleicher Konfiguration am C15 wie PGF2α mit den erwünschten pharmakologischen Eigenschaften.

Bisher wurde die 15-Hydroxygruppe durch Reduktion des 15-Ketons dargestellt, wobei als Reduktionsmittel $NaBH_4$, $NaBH_4/CeCl_3$, $Zn(BH_4)_2$ dienten (EP-A-268548).

Die oben genannten Reduktionsmittel haben jedoch den Nachteil, daß sie Gemische an 15-Hydroxyepimeren liefern. Andere Reduktionsmittel, wie S-Binal-H geben zwar hohe Stereoselektivität zugunsten des 15α-Hydroxy-epimeren, sind aber teurer, nur bei tiefen Temperaturen (-100 bis -120 °C) zu verwenden und müssen obendrein in hohem Überschuß eingesetzt werden.

Gelegentlich ist die Stereoselektivität der Reduktion an die Art der Schutzgruppe am C-11 gebunden. Z.B. wurde mit einer p-Phenylphenyl-carbamoylgruppe und einem sterisch gehinderten Lithium- oder Kaliumtrialkylhydridoborat bei -130 °C ein 15α/15β-Verhältnis von 92:8 erzielt.

Ein anderes Reduktionsmittel ist Diisobutylaluminium-2,6-di-tert.-butyl-4-methyl-phenoxid. Es kann benutzt werden, um die 15-Ketoreduktion in verschiedenen Prostaglandinen und Prostacyclinen durchzuführen.

EP 234 158 nennt zwar als mögliches Reduktionsmittel für Isocarbacyclin-Zwischenprodukte Diisobutylaluminium-2,6-ditert.butyl-4-methyl-phenoxid, es wird jedoch kein einziges Ausführungsbeispiel in dieser Anmeldung genannt. E.J.Corey et. al, JACS 106, 3875(1984) und W.Bartmann et al., Ann. 321(1987) erzielten nur mit einem hohen Reagenz-Überschuß mit bis 10 Equivalenten, der jedoch die Aufarbeitung erschwert, hohe Ausbeuten an 15α-Hydroxyepimeren aus unhalogenierten Enonen. Um die hohen Stereoselektivitäten zu erzielen, muß jedoch in 11-Stellung eine Hydroxygruppe vorhanden sein.

Eine freie 11-Hydroxygruppe stellt aber einen synthetischen Nachteil dar, weil sie bei weiteren Reaktionen zum Aufbau des Prostacyclins störend sein kann.

Wie die Tabelle 1 zeigt, ergibt die 15-Ketoreduktion an Verbindungen mit geschützter 11-Hydroxygruppe wesentlich geringere Stereoselektivitäten.

Tabelle 1

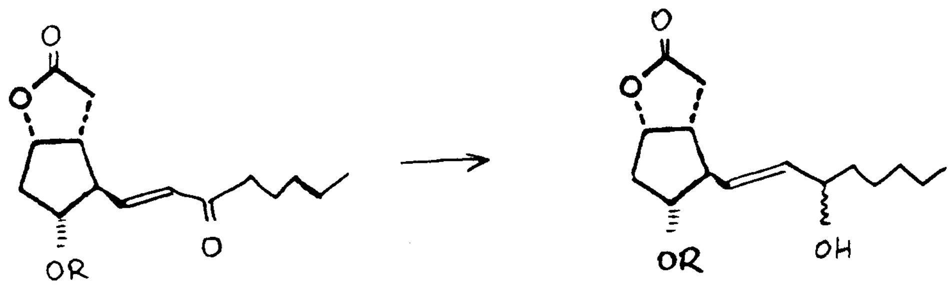

| Keton | Equivalente Dibah/Ionol | 15α/15β |
|---|---|---|
| R = H | 10 | 91 : 9 |
| R = Ac | 10 | 50 : 50 |
| R = THP | 10 | 66 : 34 |
| R = φ-φ-C=O | 10 | 50 : 50 |

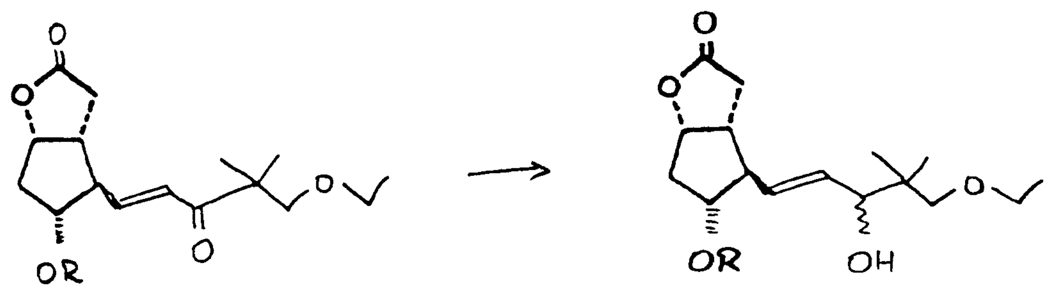

| Keton | Equivalente Dibah/Ionol | 15α/15β |
|---|---|---|
| R = H | 6 | 99 : 1 |
| R = φ-φ-C=O | 6 | 61 : 39 |

φ-φ-C=O = p-Phenyl-benzoyl

Es wurde nun gefunden, daß die in 11-Stellung mit Silylethern (TBDMS = tert.-butyldimethylsilyloxy oder TBDPS = tert.butyldiphenylsilyloxy) geschützten α-Halogenenone (1), (2), (3), (4), (5), (6) mit 1,1 bis 1,5 Equivalenten Diisobutylaluminium-2,6-ditert.butyl-4-methyl-phenoxid in Toluol bei -70 °C bis -80 °C in vier Stunden in hohen Ausbeuten von ca 80 % d. Th. und hoher Stereoselektivität (Epimerenverhältnis 92,7 - 97,4 %) zu den 15α-Hydroxyepimeren reduziert werden, wobei der nur geringe Reagenzienüberschuß eine

einfache Aufarbeitung ermöglicht.

Die nachfolgende tabellarische Übersicht stellt das erfindungsgemäße Verfahren unter Beweis:

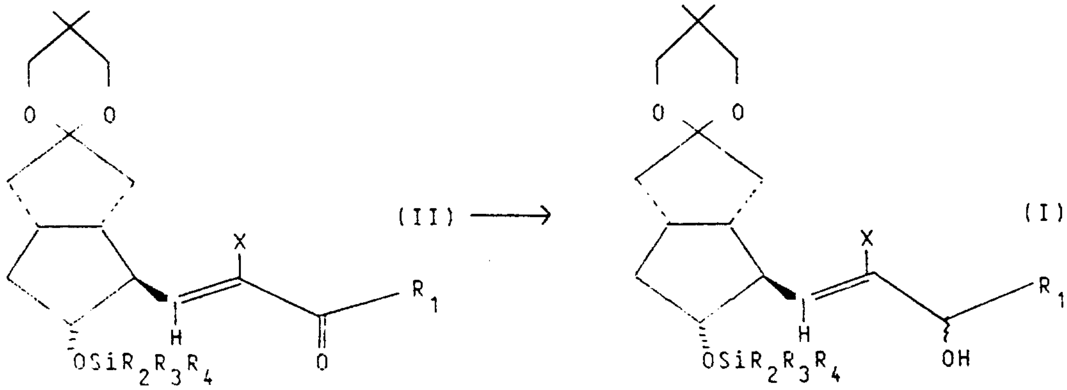

(II) (I)

Tabelle 2.

| Keton Nr. | X | R2 | R3 | R4 | R1 | Alkohol Nr. | 15α/15β |
|---|---|---|---|---|---|---|---|
| (1) | Br | t-Bu | Me | Me | 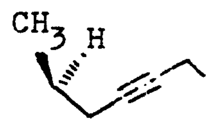 | (7) | 97.4:2.6 |
| (2) | Br | t-Bu | Me | Me | 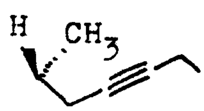 | (8) | 92.7:7.3 |
| (3) | Cl | t-Bu | Me | Me | 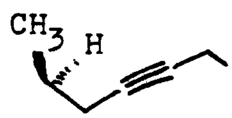 | (9) | 97.0:3.0 |
| (4) | Cl | t-Bu | Me | Me | 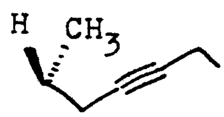 | (10) | 96.4:3.6 |
| (5) | Br | t-Bu | Me | Me | 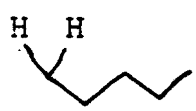 | (11) | 88.0:12.0 |
| (6) | Br | t-Bu | ϕ | ϕ | 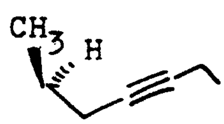 | (12) | 92.6;7.4 |

ϕ = Phenyl

Dagegen werden die nicht halogenierten, in Position 11 geschützten Ketone (13), (14), (15) und (16) auch mit 20 Equivalenten des gleichen Reduktionsmittels **bei -78°C** nur zu ca. 10 % und ohne jede Stereoselektivität reduziert.

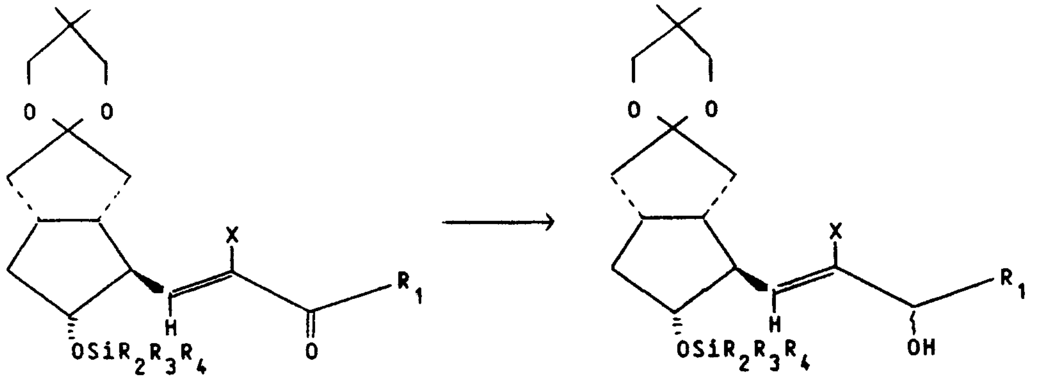

| Keton Nr. | X | R2 | R3 | R4 | R1 | 15α/15β |
|---|---|---|---|---|---|---|
| (13) | H | t-Bu | Me | Me | $CH_3$ | 50:50 |
| (14) | H | t-Bu | Me | Me | $C_5H_{11}$ | 50:50 |
| (15) | H | t-Bu | Me | Me | 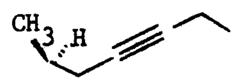 | 50:50 |
| (16) | H | t-Bu | Me | Me | 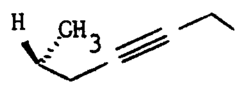 | 50:50 |

Die erfindungsgemäße stereoselektive Reduktion wird bei niedrigen Temperaturen von -40 °C bis -100 °C, vorzugsweise bei -70 bis -80 °C vorgenommen. Das Verdünnungsmittel für die Reaktion ist Toluol, andere Lösungsmittel, wie Tetrahydrofuran, Ether, Hexan, Dimethoxyethan verringern die Stereoselektivität.

Die Reduktion erfolgt vorzugsweise mit einem geringen Überschuß an Diisobutylaluminium-2,6-di-tert.-butyl-4-methyl-phenoxid (1.1-1.5 Equivalente), um eine vollständige Reaktion zu gewährleisten.

Die Reaktionszeit beträgt zwischen 1 und 12 Stunden, vorzugsweise 2-4 Stunden. Die Aufarbeitung erfolgt zweckmäßigerweise durch Alkoholyse des Aluminats mit Isopropanol und anschließende Zersetzung mit Wasser, Absaugen vom Aluminiumhydroxidniederschlag, Trocknen und Einengen der organischen Phase im Vakuum.

Die Herstellung der Verbindung (1) ist bekannt (EP 268546), die Herstellung der Verbindungen (2) bis (6) erfolgte in Analogie dazu.

Die folgenden Beispiele erläutern die Herstellung der α-Halogenenone der Formel II.

Beispiel 1

(1S,2S,3R,5R)-2-[(Z)-(4S)-2-Brom-4-methyl-3-oxo-1-nonen-6-inyl]-3-tert.butyl-dimethylsilyloxy-7,7-(2,2-dimethyl-tri-methylendioxy)-bicyclo-[3.3.0]-octan (1)

**Herstellung :**

1. In einem 1 l Dreihalskolben mit Tropftrichter, KPG-Rührer und Innenthermometer werden unter Stickstoff 4,48 g Natriumhydrid (50% in Weißöl) vorgelegt und eine Mischung von 93,5 ml THF und 16,5 ml Toluol zugetropft.
2. Die Suspension wird auf 2 °C abgekühlt und eine Lösung von 22,97 g (3S)-3-Methyl-2-oxo-5-octinyl-phosphonsäure-dimethylester in 93,5 ml THF und 16,5 ml Toluol so zugetropft, daß die Temperatur nicht über 8 °C steigt.
3. Es werden 1,3 ml Tetraisopropylorthotitanat zugegeben und 45 Minuten bei 1-2 °C nachgerührt.
4. 20,72 g N-Bromsuccinimid werden auf einmal zugegeben und 45 Minuten bei 1-2 °C nachgerührt.

5. Eine Lösung von 27,5 g (1S,2S,3R,5R)-7,7-(2,2-Dimethyl-tri-methylendioxy)-3-tert.butyldimethylsilyloxy-bicyclo-[3.3.0]-octan-carbaldehyd in 187 ml abs. THF und 33 ml Toluol wird innerhalb von 15 Minuten zugetropft.
6. Die Reaktionsmischung wird unter Eiskühlung 3 Stunden gerührt, dann über 16 Stunden auf ca. 15 °C erwärmt.
7. Die Reaktionsmischung wird mit einigen Tropfen Eisessig auf pH7 eingestellt und am Rotationsverdampfer auf ein Volumen von ca. 100 ml eingeengt.
8. Der Rückstand wird in 200 ml Wasser und 200 ml Methyl-tertbutylether aufgenommen, die wässrige Phase auf 800 ml verdünnt und dreimal mit je 150 ml Methyl-tertbutylether extrahiert.
9. Die Methyl-tertbutylether-Extrakte werden mit 100 ml Wasser gewaschen und über 80 g Natrium-sulfat getrocknet.
10. Das Natriumsulfat wird abfiltriert und mit ca. 50 ml Methyl-tertbutylether nachgewaschen.
11. Es wird eingeengt und der Rückstand an 2000 g Kieselgel mit Hexan/Essigester 100:0 bis 80:20 chromatographiert.

Ausbeute: 30,73 g = 72,6 % der Theorie.
Eigenschaften: farbloses Öl

| $[\alpha]^{20}$ (c = 1, $CHCl_3$) | | | | |
|---|---|---|---|---|
| 589 | 578 | 546 | 436 | 365 nm |
| +19.7 | +20.7 | +24.2 | +48.0 | +86.5 ° |

| Analyse: | C | H | Br | % |
|---|---|---|---|---|
| ber. | 61.36 | 8.34 | 14.08 | |
| gef. | 61.46 | 8.04 | 13.90 | |

Analog wurden die α-Halogen-enone (2), (3), (4), (5) und (6) hergestellt:

Beispiel 2

(1S,2S,3R,5R)-2-[(Z)-(4R)-2-Brom-4-methyl-3-oxo-1-nonen-6-inyl]-3-tert.butyldimethylsilyloxy-7,7-(2,2-dimethyl-tri-methylendioxy)-bicyclo-[3.3.0]-octan (2)

| Analyse | C | H | Br | % |
|---|---|---|---|---|
| ber. | 61.36 | 8.34 | 14.08 | |
| gef. | 61.34 | 8.11 | 13.85 | |

$[\alpha]_D^{20} = -4.4$ (c=1, $CHCl_3$)

Beispiel 3

(1S,2S,3R,5R)-2-[(Z)-(4S)-2-Chlor-4-methoxy-3-oxo-1-nonen-6-inyl]-3-tert.butyldimethylsilyloxy-7,7-(2,2-dimethyl-tri-methylendioxy)-bicyclo-[3.3.0]-octan (3)

| Analyse | C | H | Cl | % |
|---|---|---|---|---|
| ber. | 66.57 | 9.05 | 6.78 | |
| gef. | 66.76 | 8.95 | 6.78 | |

$[\alpha]_D^{20}$ = +11.1 (c=1, $CHCl_3$)

Beispiel 4

(1S,2S,3R,5R)-2-[(Z)-(4R)-2-Chlor-4-methyl-3-oxo-1-nonen-6-inyl]-3-tert.butyldimethylsilyloxy-7,7-(2,2-dimethyl-tri-methylendioxy)-bicyclo-[3.3.0]-octan (4)

| Analyse | C | H | Cl | % |
|---|---|---|---|---|
| ber. | 66.57 | 9.05 | 6.78 | |
| gef. | 66.09 | 8.38 | 7.38 | |

$[\alpha]_D^{20}$ = -5.0 (c=1, $CHCl_3$)

Beispiel 5

(1S,2S,3R,5R)-2-[(E)-2-Brom-3-oxo-1-octenyl-3-tert.butyl-dimethylsilyloxy-7,7-(2,2-dimethyl-tri-methylendioxy)-bicyclo-[3.3.0]-octan (5)

| Analyse | C | H | Br | % |
|---|---|---|---|---|
| ber. | 59.65 | 8.71 | 14.70 | |
| gef. | 59.94 | 8.40 | 14.34 | |

$[\alpha]_D^{20}$ = +4,5 (c=1, $CHCl_3$)

Beispiel 6

(1S,2S,3R,5R)-2-[(Z)-(4S)-2-Brom-4-methyl-3-oxo-1-nonen-6-inyl]-3-tert.butyldiphenylsilyloxy-7,7-(2,2-dimethyl-tri-methylendioxy)-bicyclo-[3.3.0]-octan (6)

| Analyse | C | H | Br | % |
|---|---|---|---|---|
| ber. | 67.71 | 7.43 | 11.54 | |
| gef. | 67.61 | 6.93 | 11.19 | |

$[\alpha]_D^{20}$ = +48° (c=1, $CHCl_3$)

Die Durchführung des erfindungsgemäßen Verfahrens wird illustriert durch die folgenden Beispiele:

Beispiel 7

(1S,2S,3R,5R)-2-[(Z)-(3S,4S)-2-Brom-3-hydroxy-4-methyl-1-nonen-6-inyl]-3-tert.-butyl-dimethylsilyloxy-7,7-(2,2-dimethyl-tri-methylendioxy)-bicyclo-[3.3.0]-octan (7)

Herstellung:

1. In einem 500 ml Dreihalskolben mit Tropftrichter, KPG-Rührer und Innenthermometer werden unter Stickstoff 65.46 ml Dibah-T (20% in Toluol) vorgelegt.
2. Es wird auf 2 °C abgekühlt und eine Lösung von 18.46 g Ionol in 50 ml Toluol so zugetropft, daß die Temperatur nicht über 4 °C steigt.
3. Es wird 45 min nachgerührt und auf -75 °C abgekühlt.
4. Eine Lösung von 29,73 g (1S,2S,3R,5R)-2-[(Z)(4S)-2-Brom-4-methyl-3-oxo-1-nonen-6-inyl]-3-tert.butyl-dimethylsilyloxy-7,7-(2,2-dimethy-tri-methylendioxy)-bicyclo-[3.3.0]-octan in 60 ml Toluol wird innerhalb von 75 min so zugegeben, daß die Temperatur nicht über -70 °C steigt.
5. Die orangefarbene Lösung wird für 45 min bei -70 °C bei -75 °C nachgerührt.
6. Es werden tropfenweise 12 ml Isopropanol zugesetzt und das Kühlbad entfernt.
7. Bei -20 °C werden vorsichtig tropfenweise 40 ml ges. Natriumbicarbonatlösung zugegeben.
8. Es wird 30 min nachgerührt und die entstandene weiße Suspension in einer G4-Fritte abgesaugt, über Natriumsulfat getrocknet, abgesaugt und bis zur Gewichtskonstanz eingeengt.
10. Der Rückstand wird an 2000 g Kieselgel mit Toluol/Essigester 100:0 bis 90:10 chromatographiert.

Ausbeute: 24,32 g = 81,5 % d. Th.
Eigenschaften: farbloses Öl

| [α] (c = 1, $CHCl_3$) | | | | |
|---|---|---|---|---|
| 589<br>+23.6 | 578<br>+24.3 | 546<br>+28.3 | 436<br>+53.8 | 365 nm<br>+98.1 ° |

| Analyse: | C | H | Br | % |
|---|---|---|---|---|
| ber. | 61.14 | 8.67 | 14.02 | |
| gef. | 61.36 | 8.28 | 13.40 | |

Analog wurden die folgenden Alkohole (8), (9), (10), (11), (12) hergestellt:

Beispiel 8

(1S,2S,3R,5R)-2-[(Z)-(3S,4R)-2-Brom-3-hydroxy-4-methyl-1-nonen-6-inyl]-3-tert.butyl-dimethylsilyloxy-7,7-(2,2-dimethyl-tri-methylendioxy)-bicyclo-[3.3.0]-octan (8)

| Analyse | C | H | Br | % |
|---|---|---|---|---|
| ber. | 61.14 | 8.67 | 14.03 | |
| gef. | 61.64 | 8.66 | 14.16 | |

$[\alpha]_D^{20} = -15.0$ (c=1, $CHCl_3$)

Beispiel 9

(1S,2S,3R,5R)-2-[(Z)-(3S,4S)-2-Chlor-3-hydroxy-4-methyl-1-nonen-6-inyl]-3-tert.butyl-dimethylsilyloxy-7,7-(2,2-dimethyl-tri-methylendioxy)-bicyclo-[3.3.0]-octan (9)

| Analyse | C | H | Cl | % |
|---|---|---|---|---|
| ber. | 66.32 | 9.40 | 6.75 | |
| gef. | 66.64 | 9.25 | 6.60 | |

$[\alpha]_D^{20} = +16.4$ (c=1, $CHCl_3$)

Beispiel 10

(1S,2S,3R,5R)-2-[(Z)-(3S,4R)-2-Chlor-3-hydroxy-4-methyl-1-nonen-6-inyl]-3-tert.butyl-dimethylsilyloxy-7,7-(2,2-dimethyl-tri-methylendioxy)-bicyclo-[3.3.0]-octan (10)

| Analyse | C | H | Cl | % |
|---|---|---|---|---|
| ber. | 66.32 | 9.40 | 6.75 | |
| gef. | 66.43 | 9.25 | 6.78 | |

$[\alpha]_D^{20} = +30.2$ (c=1, $CHCl_3$)

Beispiel 11

(1S,2S,3R,5R)-2-[(Z)-(3S)-2-Brom-3-hydroxy-1-octenyl]-3-tert.butyl-dimethylsilyloxy-7,7-(2,2-dimethyl-tri-methylendioxy)-bicyclo-[3.3.0]-octan (11)

| Analyse | C | H | Br | % |
|---|---|---|---|---|
| ber. | 59.44 | 9.05 | 14.41 | |
| gef. | 59.70 | 8.61 | 14.08 | |

$[\alpha]_D^{20} = +9.4$ (c=1, $CHCl_3$)

Beispiel 12

(1S,2S,3R,5R)-2-[(Z)-(3S,4S)-2-Brom-4-methyl-3-oxo-1-nonen-6-inyl]-3-tert.butyl -diphenylsilyloxy-7,7-(2,2-dimethyl-tri-methylendioxy)-bicyclo-[3.3.0]-octan (12)

| Analyse | C | H | Br | % |
|---|---|---|---|---|
| ber. | 67.51 | 7.70 | 11.52 | |
| gef. | 67.47 | 7.62 | 11.05 | |

$[\alpha]_D^{20} = +46.0$ (c=1, $CHCl_3$)

Das pharmakologisch wirksame Cicaprost wird nach W. Skuballa et al. (J.Med.Chem. 1986, Vol.29, 313) aus (1S,2S,3R,5R)-3-Hydroxy-2-[(3S,4S)-3-hydroxy-4-methyl-1,6-nonadiinyl]-bicyclo[3.3.0]octan-7-on hergestellt, das man aus den Verbindungen der Formel I durch Basen-katalysierte HX-Eliminierung und anschließende Säure-katalysierte Abspaltung der Schutzgruppen (Ketal, Silylether) erhält. Die beiden letzten Verfahrensschritte sind literaturbekannt.

## Patentansprüche

**1.** Verfahren zur Herstellung von (1S, 2S, 3R,5R)-2-[(3S)-2-Halogen-3-hydroxy-1-alken(inyl)]-3-trialkylsilyloxy-7,7-(2,2-dimethyl-trimethylendioxy)-bicyclo-[3.3.0]-octan-Verbindungen der allgemeinen Formel I

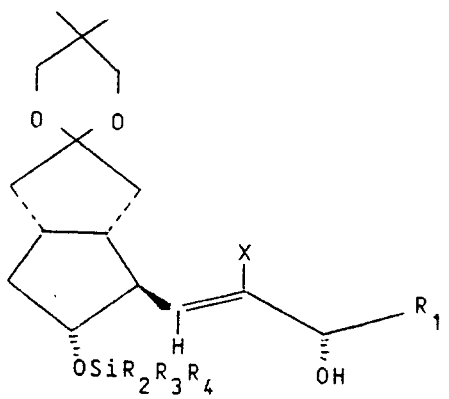

(I),

worin

| | |
|---|---|
| X | Chlor oder Brom, |
| $R_1$ | geradkettiges oder verzweigtes Alkyl mit bis zu 6 C-Atomen, geradkettiges oder verzweigtes Alkenyl mit bis zu 6 C-Atomen, geradkettiges oder verzweigtes Alkinyl mit bis zu 6 C-Atomen, |
| $R_2$, $R_3$, $R_4$ | jeweils gleich oder verschieden sein können und $C_1$-$C_4$-Alkyl und ggf. durch $C_1$-$C_4$-Alkylgruppen substituiertes Phenyl bedeuten können, wobei die 13,14-Doppelbindung bezüglich der C-Kette die trans-Konfiguration aufweist, |

durch stereoselektive Reduktion eines $\alpha$-Halogenen-enons der allgemeinen Formel II

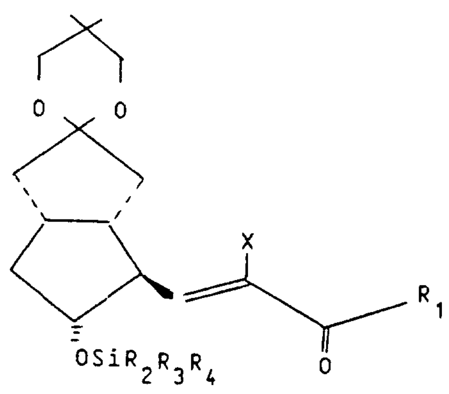

(II),

worin X, $R_1$, $R_2$, $R_3$, $R_4$ die vorstehend angegebene Bedeutung haben, mit einem aus Diisobutylaluminiumhydrid und 2,6-Di-tert.-butyl-4-methyl-phenol hergestellten Reagenz.

## Claims

**1.** A process for the preparation of (1S,2S,3R,5R)-2-[(3S)-2-halo-3-hydroxy-1-alken(ynyl)]-3-trialkylsilyloxy-7,7-(2,2-dimethyltrimethylenedioxy)bicyclo[3.3.0]octane compounds of general formula I

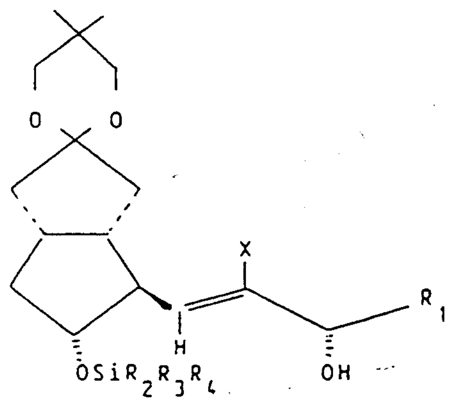

(I)

wherein

X may be chlorine or bromine,

$R_1$ may be straight-chain or branched alkyl having up to 6 carbon atoms, straight-chain or branched alkenyl having up to 6 carbon atoms, or straight-chain or branched alkynyl having up to 6 carbon atoms,

$R_2$, $R_3$, $R_4$ may in each case be identical or different and may be $C_1$-$C_4$alkyl, or phenyl optionally substituted by $C_1$-$C_4$alkyl groups,

the 13,14-double bond having the trans-configuration with respect to the carbon chain, by stereoselective reduction of an α-halo-enone of general formula II

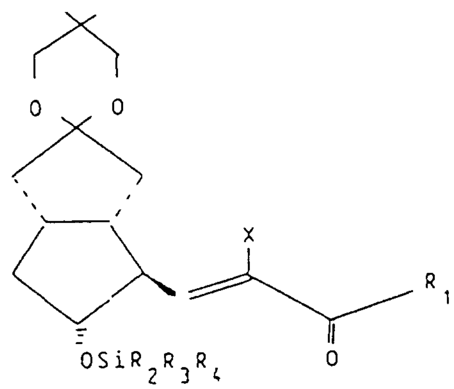

(II)

wherein X, $R_1$, $R_2$, $R_3$, $R_4$ have the meanings given above, with a reagent produced from diisobutylaluminium hydride and 2,6-di-tert-butyl-4-methylphenol.

## Revendications

1. Procédé de préparation de (1S, 2S, 3R, 5R)-2-[(3S)-2-halogéno-3-hydroxy-1-alcén(ynyl)]-3-trialkylsilyloxy-7,7-(2,2-diméthyl-triméthylène-dioxy)-bicyclo-[3.3.0]-octanes de formule générale I ci-dessous :

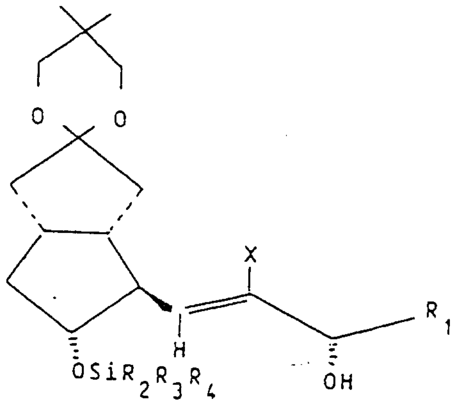

(I)

(dans laquelle

X désigne le chlore ou le brome,

$R_1$ un alkyle à chaîne linéaire ou ramifiée pouvant avoir jusqu'à 6 atomes de carbone, un alcényle linéaire ou ramifié pouvant avoir jusqu'à 6 atomes de carbone ou un alcynyle linéaire ou ramifié pouvant avoir jusqu'à 6 atomes de carbone, et

$R_2$, $R_3$ et $R_4$, qui peuvent être identiques ou différents les uns des autres, peuvent être des alkyles en $C_1$-$C_4$ ou des phényles éventuellement substitués par des alkyles en $C_1$-$C_4$, la double-liaison en 13,14 ayant la configuration trans relativement à la chaîne carbonée),

par réduction stéréosélective d'une α-halogéno-énone de formule générale II :

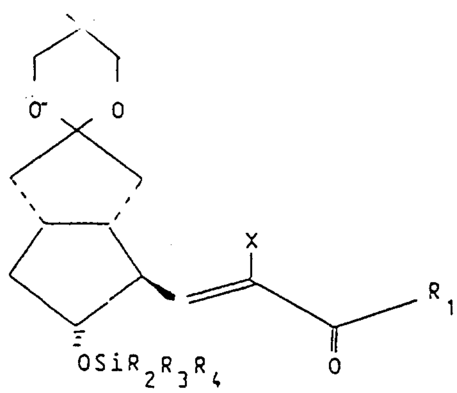

(II)

les divers symboles ayant les significations ci-dessus indiquées, avec un réactif obtenu à partir d'hydrure de diisobutylaluminium et de 2,6-di-tert-butyl-4-méthylphénol.